# EUROPÄISCHE PATENTSCHRIFT

(11) **EP 0 850 631 B1**
(45) Veröffentlichungstag und Bekanntmachung des Hinweises auf die Patenterteilung: **05.03.2003**
(21) Anmeldenummer: 96120985.5
(22) Anmeldetag: 30.12.1996
(51) Int. Cl.: A61F 13/42, G09B 19/00

(54) **Anordnung zum Anzeigen von auftretender Feuchtigkeit**
Wetness indicating device
Dispositif d'indication d'humidité

(43) Veröffentlichungstag der Anmeldung: 01.07.1998
(73) Patentinhaber: Boscaro, Gianluca, 6992 Cimo (CH)
(72) Erfinder: Boscaro, Gianluca, 6992 Cimo (CH)
(74) Vertreter: Faggioni, Giovanmaria

(56) Entgegenhaltungen:
- WO-A-95/15739
- WO-A-96/25904
- BE-A- 902 427
- FR-A- 2 582 812
- FR-A- 2 587 616

## Beschreibung

Die vorliegende Erfindung betrifft eine Anordnung zum Anzeigen von auftretender Feuchtigkeit, sowie eine mit dieser Anordnung versehene Windel, die besonders nützlich für Pflegetätigkeiten sind.

Ältere Personen, welche eine Unterleibsoperation hinter sich haben, wie beispielsweise eine Prostataoperation, leiden unter sogenannter Inkontinenz. So ist es möglich, dass derartige Personen teilweise oder gänzlich bettlägerig sind und/oder mit Einschränkungen der Mobilität und der Bewegungsfreiheit konfrontiert sind, wobei sehr oft eine reduzierte Kontrollfähigkeit der Harnleiter-Schliessmuskeln hinzukommt. Die ungenügende Kontinenzfähigkeit des Urins erzwingt beispielsweise im Rahmen der Pflegetätigkeit in Spitälern die Verwendung von Blasenkathetern, von Kondomen, Windeln und dergleichen, sowohl für sich allein oder in Kombination miteinander. Alle diese heute zur Anwendung gelangenden Lösungen, wie insbesondere die Verwendung eines Katheters, haben den Nachteil, dass Infektionen an den Harnwegen entstehen können, und zwar Infektionen, die nicht nur lokal auftreten, sondern sich in den Harnwegen verbreiten. Diese Infektionen führen normalerweise zu einer Verschlechterung des allgemeinen Zustandes des Patienten. Die erwähnten Komplikationen entstehen praktisch immer, auch wenn man mit aller Sorgfalt die Auswechslung und/oder die Abnahme eine Katheters vornimmt.

Die Verwendung eines Kondomes hat den Nachteil, dass an den abgedeckten Teilen Verfaulungserscheinungen auftreten können.

Die Verwendung von Windeln bzw. von sogenannten Hosenwindeln,

Wie beispielweise Pampers® bergen hygienische Probleme in sich, was zu Entzündungen, Hautreizungen bzw. zur Entstehung von Dekubituswunden führen kann, wenn man nicht innerhalb einer angemessenen Zeitspanne die Windeln ersetzt.

Die Verhinderung der erwähnten Probleme ist nur möglich durch sehr häufige Kontrolle durch das Pflegepersonal, was aufwendig ist und eine umfangreiche Reinigungsarbeit mit sich bringt, indem grosse Wäschemengen zu bewältigen sind. Zudem ist auch bei häufiger Kontrolle nicht sichergestellt, dass bereits unmittelbar nach erfolgter Kontrolle ein Patient bereits wieder nass liegt.

Einige Lösungen des letzgenannten Problemes sind kürzlich vorgeschlagen worden.

WO 95/15 739 schlägt eine Anordnung zum Anzeigen von auftretender Feuchtigkeit vor, die in Form einer Kleidung ist und die mindestens teilweise als Unterhose gebildet ist; sie enthält einen Detektor von Feuchtigkeit, welcher mit einem Alarm verbunden ist. Der Alarm enthält ein Signalübertragungsgerät, das ein Alarmsignal zu einem getrennten Empfänger überträgt, um eine Fernanzeigung von Notzustand abzugeben.

WO 96/25 904 beschreibt eine Anordnung zum Erfassen und Anzeigen der Feuchtigkeit, wobei sie zwei Leiter umfaßte die Teil eines Sensorstreifens sind. Der Streifen hat auf ihrer Oberflache ein Leiter und ist aus einem nicht-leitend Material hergestellt. Der Streifen soll an einer Unterhose durch eine Schicht von Klebstoff geklebt werden.

BE-A-902 427 beschreibt einen Detektor, der an einer Windel aufgebracht ist, und der abgetrennt und verbunden werden kann. Sie betrifft auch eine in ihrer internen Struktur modifizierte Windel, die einen im absorbierendem Material eingebetteten, das Körper der Person nicht berührenden Sensor umfasst.

FR-A-2 582 812 beschreibt einen Detektor von Feuchtigkeit, der trennbar ist und der eine obere wasserdurchlässige Schicht, eine untere wasserundurchlässige Schicht und zwei zwischen den obengenannten Schichten gelegte metallische Schichte, wobei die letzten Schichte parallel miteinander sind und eine von den Schichten durch eine isolierende Schicht gedeckt ist. Die obere Schicht ist aus Baumwolle, Sojapapier odgl.. Die untere Schicht is aus einem Kunststoffmaterial.

FR-A-2 587 616 beschreibt eine Einrichtung, die zwei an einer wasserundurchlässigen aus Kunststoff gebildeten Schicht Metalschichte aufweist Die Metalschichte sind an verschiedenen Seiten der Schicht aufgebracht und sie sind elektrisch an einer Signalvorrichtung verbunden.

Es ist daher eine Aufgabe der vorliegenden Erfindung, eine Massnahme vorzuschlagen, mittels welchen die obenerwähnten Probleme gelöst oder mindestens gelindert werden können und Personen, welche eine ungenügende Kontinenzfähigkeit des Urins besitzen, vor den äusserst unangenehmen Begleiterscheinungen zu verschonen.

Die erwähnte Aufgabe wird durch eine Anordnung zum Anzeigen von auftretender Feuchtigkeit gelöst die einen Sensor umfaßt, der die Feuchtigkeit erfassen kann, wobei der Sensor ein Papier, einen unter dem Papier angeordneten, aus einem Polymer bestehenden Streifen, und zwei beidseits des Streifens angeordnete Kupferdrähte aufweist, dadurch gekennzeichnet, daß der Sensor ein mit Silikon behandeltes, auf der dem Papier gegenüber entgegengesetzten Seite des Sensors und auf dem aus Polymer bestehenden Streifen angeordnetes Papier aufweist, und daß das verwendete Polymer ein hydrophobes Polymer ist.

Bevorzugsweise ist das Papier weisses Papier und das hydrophobe Polymer Polypropylen.

Die vorliegende Erfindung betrifft auch eine mit dem obengenannten Sensor versehene Windel.

Vorgeschlagen wird eine Anordnung zum Anzeigen von auftretender Feuchtigkeit, wie insbesondere von Urinnässe, welche einerseits einen Sensor aufweist mit beabstandet angeordneten elektrischen Leiterdrähten, welche auf einem die Feuchtigkeit bzw. den Urin an- bzw. aufnehmenden Substrat angeordnet sind, sowie einen elektrischen Schaltkreis, welcher mit den Leiterdrähten verbunden ist und diese mittels eines Stromkreises beaufschlagt und welcher Schaltkreis, aufgrund einer Impedanz bzw. abnehmendem Widerstand bis hin zu einem Kurzschluss zwischen den beiden Drähten infolge zwischen diesen am bzw. auf dem Substrat auftretender Feuchtigkeit bzw. aufgenommenen Urins, eine Anzeige auslöst.

Die beiden Leiterdrähte sind beispielsweise über ein Kabel mit dem Schaltkreis verbunden und werden von diesem mit einem Schwachstromkreis beaufschlagt, wobei bei auftretender Feuchtigkeit im Bereich des Sensors die oder der sich einstellende Impedanz bzw. Kurzschluss zwischen den Leitern eine Anzeige auslöst, wie beispielsweise ein akustisches, ein visuelles Signal oder ein Bewegungssignal, wie beispielsweise eine Vibrationsanzeige, welche auf die auftretende Feuchtigkeit bzw. den ausgetretenen Urin hinweisen.

Es ist möglich, mittels der erfindungsgemäss definierten Anordnung auftretende Feuchtigkeit in Räumlichkeiten zu detektieren, sei dies in kellerartigen Räumlichkeiten, wo infolge Grundwasser oder Bergdruck erhöhte Feuchtigkeit oder sogar Wassereinbrüche auftreten können, oder aber in Wohnungen, um unkontrollierten Über- bzw. Auslaufen von Flüssigkeiten zu verhindern. So ist es möglich, das Auslaufen von Wasser aus Röhren oder aus offengelassenen Wasserhahnen zu detektieren, indem ein beispielsweise im Fussboden angeordneter erfindungsgemässer Sensor die auftretende Feuchtigkeit aufgrund der Impedanz bzw. des Kurzschlusses erfasst und über den damit verbundenen Schaltkreis eine Anzeige, wie beispielsweise eine Sirene, auslöst.

Auch ist es möglich, bei der Füllung eines Behälters das Erreichen eines bestimmten Niveaus zu detektieren oder zu verhindern, dass das zu füllende Behältnis überläuft. So kann der Sensor auf der gewünschten Höhe im Innern des Behälters angebracht werden. Weiter ist es sogar möglich, zum Beispiel wahllos vom Schaltkreis mittels eines Impulses einen Schliessmechanismus zu betätigen, um das weitere Füllen des Behälters zu unterbrechen.

Schlussendlich ist es aber auch möglich, mittels der erfindungsgemässen Anordnung anzuzeigen, wenn beispielsweise ein Bauwerk ausgetrocknet ist. Sobald der im Bauwerk angeordnete Sensor keine Impedanz oder keinen Kurzschluss zwischen den Leiterdrähten mehr detektiert, kann davon ausgegangen werden, dass der Sensor trocken ist und damit verbunden selbstverständlich auch das Bauwerk.

Die oben angeführten Beispiele sollen zeigen, dass mittels der erfindungsgemäss definierten Anordnung grundsätzlich jegliche Art von Feuchtigkeit bzw. von auftretendem Wasser detektiert werden kann.

Die Erfindung wird nun anschliessend beispielsweise und unter Bezug auf die beigefügten Figuren näher erläutert.

Dabei zeigen:
- Fig. 1: schematisch in Perspektive einen erfindungsgemässen Sensor-Papierstreifen für das Erfassen der Feuchtigkeit,
- Fig. 2: schematisch in Perspektive ein Anzeigegehäuse, beinhaltend den elektrischen Schaltkreis,
- Fig. 3: schematisch dargestellt, Sensor, Verbindungskabel und Anzeigegehäuse, um die Funktionsweise der erfindungsgemässen Anordnung darlegen zu können,
- Fig. 4: eine weitere Ausführungsvariante der Anordnung mit einer ferngesteuert auslösbaren Anzeige und
- Fig. 5: eine Ausführungsvariante der Anordnung mit einer anderen Anschlussvariante des Schaltkreises an den Sensor.

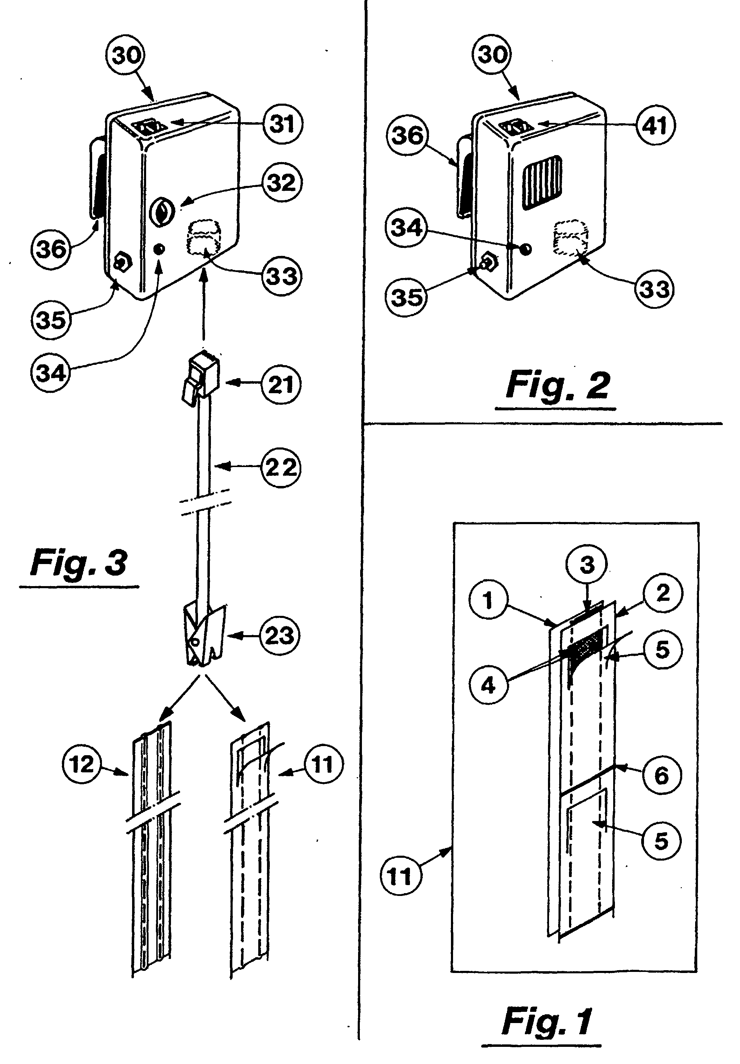

Anhand der Figuren 1 bis 5 sollen Anordnungen für die Lichtanzeige, akustische Anzeige oder Vibrationsanzeige näher erläutert werden, beispielsweise bei dem Durchnässen einer Windel durch einen Patienten, beispielsweise in einem Pflegeheim, einem Spital oder dergleichen.

Die Anordnung hat den Zweck, mit Licht, einem akustischen Signal je nach Wahl, d.h. nach Bedürfnis des Patienten oder der Pflegeorganisation anzuzeigen, dass die durch grosse oder kleine Windeln bedeckte Haut vom trockenen Zustand in einen nassen Zustand übergegangen ist, in der Regel verursacht durch Inkontinenz. Demzufolge muss dann die jeweilige Windel gewechselt werden. Dabei weist die Anordnung, wie in Fig. 1 dargestellt, einen Sensor 11 auf, welcher folgende Zusammensetzung erfindungsgemäss aufweist:
- Ein mit Silikon behandeltes Papier 1 mit einer Breite von 15 mm sowie variabler Dicke
- Auf der entgegengesetzten Seite des Sensors ist ein weisses Papier 2 aus reiner Zellulose angeordnet mit derselben Breite und wiederum mit variabler Dicke.
- Zwischen den beiden Papierstreifen ist ein Streifen 3 aus Polypropylen angeordnet mit einer Breite von 5 mm und erneut variabler Dicke.
- Beidseits des Polypropylenstreifens sind zwei Kupferdrähte 4 angeordnet, mit einem Durchmesser beispielsweise von 0,3 mm. Die Entfernung zwischen den beiden Kupferdrähten ergibt sich aus der Breite des Polypropylenstreifens.

In Fig. 1 ist an sich ein Ausschnitt aus einem abrollbaren Sensorstreifen dargestellt, welcher entlang der Linie 6 unterteilt werden kann, um so einen einzelnen Sensor vom aufgewickelten Streifen abzutrennen. Endständig am jeweiligen Streifen ist es möglich, ein eingeschnittenes U-Profil 5 vorzusehen, welches ein Anhängen des Sensors bzw. der Kupferdrähte an einer Steckdose oder an einer Klemme ermöglicht.

In Fig. 2 ist ein Anzeigegehäuse 30 dargestellt, beinhaltend einen elektrischen Schaltkreis, welcher beispielsweise mittels einer im Gehäuse angeordneten Batterie mit Strom versorgt wird. Der Schaltkreis ist über eine Steckdose 33 mit dem in Fig. 1 dargestellten Sensorstreifen verbindbar. Über eine Anzeige 34 wird die Ladung der Batterie angezeigt, d.h. beispielsweise bei Aufleuchten eines roten Lämpchens wird angezeigt, dass die Ladung der Batterie dem Ende entgegengeht. Über einen Schalter 35 ist es möglich, die Stromversorgung des Schaltkreises zu unterbrechen, beispielsweise, um damit ein Entladen der Batterie zu verhindern. Ebenfalls weist das Gehäuse einen rückseitigen Halteclips 36 auf, um das Gehäuse beispielsweise an der Hemdtasche oder einem Jakkenrevers von einer dieses Kleidungsstück tragenden Pflegeperson zu befestigen.

Schlussendlich ist an diesem Gehäuse ein akustisches oder ein visuelles Signal angeordnet, wie beispielsweise eine Sirene 31 oder eine Lampe, welche auftretende Inkontinenz anzeigt. Es ist aber auch möglich, im Gehäuse einen Vibrationsmechanismus vorzusehen, welcher aufgrund auftretender Inkontinenz ausgelöst wird und somit das Bedienungspersonal oder einen Patienten darauf aufmerksam macht.

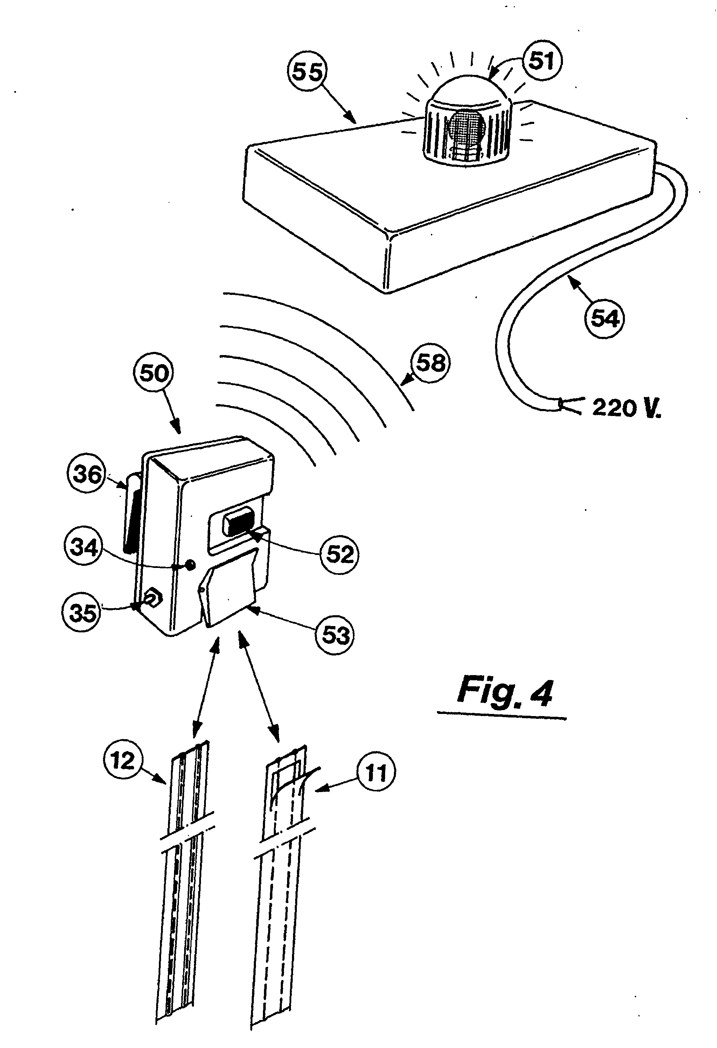

In Fig. 3 ist nun die gesamte Anordnung schematisch dargestellt, um die Funktionsweise derselben besser beschreiben zu können. Das Anzeigegehäuse 30 ist über ein Kabel 22 mit dem Sensor 11 verbunden, wobei ergänzend ebenfalls ein Sensor 12 dargestellt ist, welcher beispielsweise aus einem Textilstreifen gefertigt sein kann. Endständig wird der Sensor, d.h. der Papierstreifen 11 oder der Textilstreifen 12, mittels einer Anschlussklemme 23 verbunden, welche Anschlussklemme an die jeweiligen beiden Kupferdrähte angeschlossen wird. Auf der entgegengesetzten Seite des Verbindungskabels 22 ist ein Stecker 21 vorgesehen, welcher in die Anschlussbuchse 33 des Anzeigegehäuses 30 eingesteckt wird, um den Sensor mit dem im Gehäuse angeordneten Schaltkreis zu verbinden.

Der Papierstreifen 11 bzw. Textilstreifen 12 kann beispielsweise in einer Windel, im Bettzeug oder einem Kleidungsstück eines Patienten bzw. einer älteren Person, welche an ungenügender Kontinenzfähigkeit des Urins leidet, angeordnet werden, wobei darauf zu achten ist, dass das mit Silikon behandelte Papier auf der Haut aufgelegt werden muss, um auftretende Transpiration aufzuhalten, insbesonderen, wenn der Patient bzw. die ältere Person in einem Bett liegt, um auf diese Weise falsche Alarmsignale zu verhindern.

Damit ist das den Urin aufsaugende Papier vom Körper entfernt angeordnet, wodurch die Aufsaugefunktion verzögert wird, so dass bei Verlust von nur kleineren Mengen Urins nicht bereits ein Alarm ausgelöst wird. Ansonsten wird das Pflegepersonal bereits bei kleinsten Mengen austretenden Urins alarmiert, obwohl die Windeln noch nicht zu wechseln sind.

Der Sensor kann dabei entweder direkt in die Windel eingebaut werden bei der Herstellung der Windeln oder aber nachträglich zwischen Windel und Körperhaut eingefügt werden.

Falls nun der Patient unkontrolliert grössere Mengen an Urin abgegeben hat, entsteht ein Impedanz-Signal oder ein Kurzschluss, bzw. der zwischen den Kupferdrähten herrschende Widerstand nimmt aufgrund der Feuchtigkeit ab, was über das Verbindungskabel 22 und Stecker 33 in den Schaltkreis weitergeleitet wird. Dieses so übertragene Signal des Kurzschlusses bzw. des abnehmenden Widerstandes oder der Impedanz löst einen akustischen Alarm, ein Licht oder ein Vibrationsaggregat aus, wodurch das Pflegepersonal darauf aufmerksam gemacht wird, dass die Windel bei einem Patienten zu wechseln ist.

Der Schaltkreis ist nun so ausgelegt, dass das vom Sensor übertragene Signal nur einen relativ kurzen Impuls erzeugt, welcher den akustischen Alarm, das Licht oder das Vibrationsaggregat auslöst, währenddem letztere unter Strom bleiben, um das Pflegepersonal auf den Pflegefall aufmerksam zu machen. Der Alarm bzw. das Vibrationsaggregat werden erst ausgeschaltet zum Zeitpunkt, wo der Sensor gewechselt wird, was in der Regel mit dem Wechsel der Windeln zusammenfällt.

Der Alarm bzw. das Vibrationsaggregat können sowohl von einer nicht wiederaufladbaren Batterie oder von einer aufladbaren Batterie mit Strom versorgt werden.

In Fig. 4 ist eine weitere Ausführungsvariante der erfindungsgemässen Anordnung dargestellt.

Im Unterschied zur Anordnung in Fig. 3 erfolgt in der Anordnung gemäss Fig. 4 das Auslösen eines Signalgebers mittels Fernsteuerung bzw. mittels Funk oder drahtloser Übermittlung. Wiederum wird durch einen Sensor 11 bzw. 12 das Auftreten der Feuchtigkeit registriert bzw. es entsteht eine Impedanz oder eine Abnahme des Widerstandes zwischen den beiden Kupferdrähten, bis hin zu einem Kurzschluss, was erneut in einem Schaltkreis in einem Schaltgehäuse 50 registriert wird. Dadurch wird ein Wellenimpuls 58 ausgelöst, wobei es sich beispielsweise um Hochfrequenzwellen handeln kann, welche an einem entfernt angeordneten Anzeigegerät 55 ein akustisches oder ein visuelles Signal 51 auslösen.

Der Vorteil der Anordnung liegt darin, dass es einerseits unwichtig ist, wo der Wellenimpulsgeber bzw. das Schaltkreisgehäuse 50 angeordnet ist. Dieses kann beispielsweise unmittelbar beim Patienten bzw. in der Nähe der Sensorstreifen 11 bzw. 12 angeordnet werden, so dass praktisch auf ein Verbindungskabel 22 verzichtet werden kann. Dies geschieht, indem beispielsweise das Schaltkreisgehäuse 50 mittels einer Klammer 53 direkt an den Sensorstreifen befestigt wird. Damit die Batterie im Schaltkreisgehäuse 50 nur während jeweils sehr kurzer Zeit entladen wird, wird zum Zeitpunkt, da der Sensorstreifen nass wird und damit eine Impedanz entsteht, nur ein Impulswellensignal von einer kurzen Dauer von ca. 2 Sekunden abgegeben, worauf der Kreis in die sogenannte Standby-Position zurückgesetzt wird. Der Wellenimpuls aktiviert ein Relais im Anzeigegehäuse 55, wodurch beispielsweise eine Lampe oder eine Sirene 51 aktiviert wird, wobei dieses Alarmsignal aktiviert bleibt bis zum Wechseln der Windeln. Das Alarmsignal selbst ist beispielsweise an der normalen Stromversorgung, d.h. an einem Strom mit 220 Volt angeschlossen, so dass hier das Problem der Batterieentladung gar nicht entstehen kann.

Damit das Gerät beispielsweise in einem Spital oder einem Pflegeheim Verwendung finden kann, wo eine grössere Anzahl von Patienten zu überwachen ist, wird weiter vorgeschlagen, das Wellensignal mittels eines zusätzlichen Codes zu überlagern. Dadurch wird es möglich, an einer Anzeigetafel, wo mehrere Lampen zum Anzeigen der Nässe bei einem Patienten angeordnet sind, sofort festzustellen, bei welchem Patienten beispielsweise die Windeln zu wechseln sind.

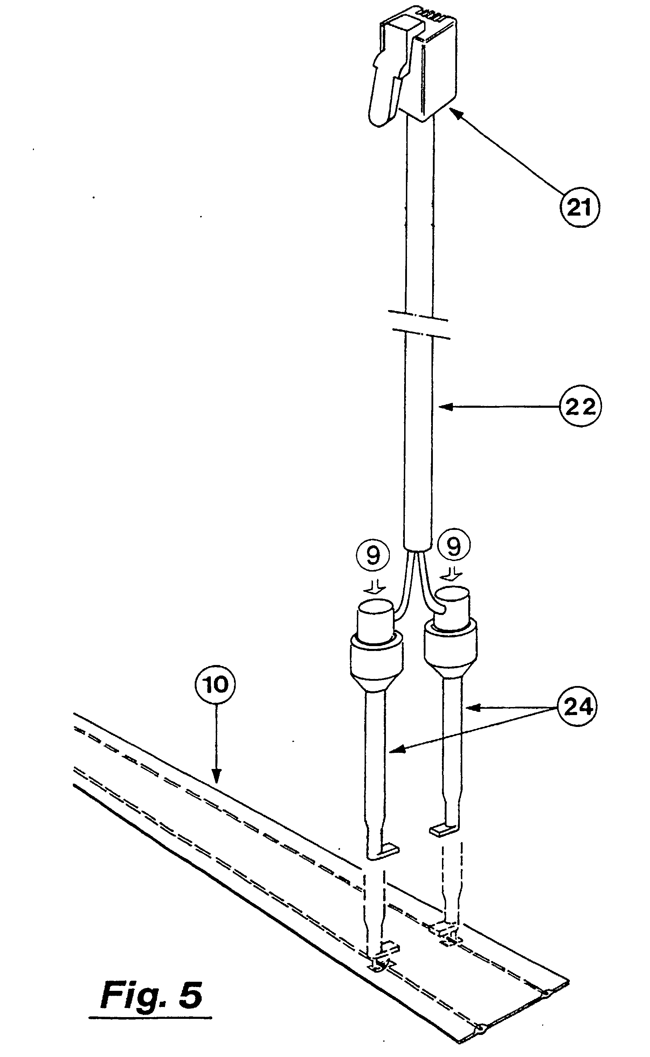

In Fig. 5 ist erneut eine erfindungsgemässe Anordnung dargestellt, jedoch aufweisend eine andere Anschlussvariante des Verbindungskabels 22 an die beiden Leiterdrähte 4 im Sensor 10. Der Anschluss erfolgt mittels zweier zangenartigen Klammern 24, welche an ihrer Frontseite mittels eines Druckknopfes 9 betätigbare zangenartige Klammern aufweisen, welche an die beiden Drähte 4, diese festklemmend angeschlossen werden können. Durch dieses festklemmende Anschliessen der beiden Anschlussklemmen 24 an die beiden Drähte 4 im Sensor 10 ist es nicht mehr notwendig, im Sensor 10 eingeschnittene U-Profile 5 vorzusehen, was natürlich die Herstellung vereinfacht.

Es versteht sich von selbst, dass der Anschluss, wie schematisch in Fig. 5 dargestellt, selbstverständlich auch in den Anordnungen dargestellt in den Fig. 3 und 4 möglich ist.

Bei den in den Figuren 1 bis 5 dargestellten erfindungsgemässen Anordnungen handelt es sich selbstverständlich nur um Beispiele, um die vorliegende Erfindung besser beschreiben zu können. Selbstverständlich ist es möglich, die dargestellte Anordnung auf x-beliebige Art und Weise abzuändern, zu modifizieren oder zu ergänzen, wesentlich ist, dass mittels eines erfindungsgemäss definierten Sensors an einer genau definierten Stelle festgestellt werden kann, dass an diesem Ort Feuchtigkeit aufgetreten ist. Insbesondere wird mittels des Sensors beispielsweise bei einem Patienten festgestellt, ob dieser unkontrolliert Urin gelöst hat, wodurch das Wechseln der Windeln notwendig wird.

## Patentansprüche

1. Anordnung zum Anzeigen von auftretender Feuchtigkeit, die einen Sensor (11) umfaßt, der die Feuchtigkeit erfassen kann, wobei der Sensor (11) ein Papier (2), einen unter dem Papier (2) angeordneten, aus einem Polymer bestehenden Streifen (3), und zwei beidseits des Streifens angeordnete Kupferdrähte (4) aufweist, **dadurch gekennzeichnet, daß** der Sensor (11) ein mit Silikon behandeltes, auf der dem Papier (2) gegenüber entgegengesetzten Seite des Sensors (11) und auf dem aus Polymer bestehenden Streifen (3) angeordnetes Papier (1) aufweist, und daß das verwendete Polymer ein hydrophobes Polymer ist.

2. Anordnung nach Anspruch 1., **dadurch gekennzeichnet, daß** das Papier (2) des Sensors (11) ein weisses Papier ist.

3. Anordnung nach Ansprüchen 1, oder 2., **dadurch gekennzeichnet, daß** das hydrophobe Polymer, aus dem der Streifen (3) des Sensors (11) hergestellt ist, Polypropylen ist.

4. Windel, an der eine Anordnung nach einem vorliegenden Anspruch angebracht ist.

## Claims

1. Device for signalling the presence of moisture comprising a sensor (11) which is apt to sense moisture, wherein the sensor (11) comprises a paper (2), a polymer strip (3) placed under the paper (2) and two copper wires placed at both sides of the strip, **characterised in that** the sensor (11) comprises a paper (1) treated with silicone placed at the side of the sensor (11) opposite to the paper (2) and on the polymer strip (3), and **in that** the polymer used is a hydrophobic polymer.

2. Device as in claim 1), **characterised in that** the paper (2) of the sensor (11) is white paper.

3. Device as in claims 1) or 2), **characterised in that** the hydrophobic polymer, of which the strip (3) of the sensor (11) is made, is polypropylene.

4. Sanitary towel to which a device as in any of the preceding claims is applied.

## Revendications

1. Dispositif d'indication de l'humiditê formée comprenant un capteur (11) qui peut détecter l'humiditë, le capteur (11) présentant un papier (2), une bande (3) réalisée dans un polymère, disposée sous le papier (2), et deux fils de cuivre (4) disposés de chaque côté de la bande, **caractérisé en ce que** le capteur (11) présente un papier (1) traité au silicone, disposé du côtê du capteur (11) opposé au papier (2) et sur la bande (3) réalisée en polymère, et **en ce que** le polymère utilisé est un polymère hydrophobe.

2. Dispositif selon la revendication 1, **caractérisé en ce que** 1e papier (2) du capteur (11) est un papier blanc.

3. Dispositif selon la revendication 1 ou la revendication 2, **caractérisé en ce que** le polymère hydrophobe dans lequel la bande (3) du capteur est réalisée, est du polypropylène.

4. Couche, sur laquelle est posé un dispositif selon une revendication précédente.
